# EUROPEAN PATENT APPLICATION

(11) **EP 1 316 943 A2**
(43) Date of publication of application: **04.06.2003**
(21) Application number: 02024936.3
(22) Date of filing: 06.11.2002
(51) Int. Cl.: G10K 11/04

(54) **An acoustic filter**

(30) Priority: 28.11.2001 JP 2001362993
(71) Applicant: Omron Corporation, Kyoto-shi, Kyoto 600-8530 (JP)
(72) Inventor: Ashida, Tameo, Omron Corp., 801, Minamifudodo-cho, Kyoto-shi, Kyoto 600-8530 (JP); Inagaki, Takashi Omron Corp. 801, Minamifudodo-cho, Kyoto-shi, Kyoto 600-8530 (JP)
(74) Representative: Kilian, Helmut, Dr.

(57) **Abstract**

The present invention provides an acoustic filter having a structure that does not affect upon the characteristics of an acoustic filter. This acoustic filter (1) is equipped with a filter case (10) having an air pressure inlet (12), a filter cap (50) having an air pressure outlet (56), and a ring-shaped packing component (80) attached to the air pressure outlet. A welding conjunction structure is employed between the filter case and the filter cap.

## Description

### BACKGROUND OF THE INVENTION

### Field of the Invention

The present invention relates to an acoustic filter, and more particulary to an acoustic filter for removing pressure noise occurring during pressurization of an air pump used in an electronic tonometer and the like.

### Description of the Background Art

First, with reference to Fig. 18, the outline structure of an electronic tonometer is explained. An electronic tonometer is roughly divided into an arm band 100, a pressure detection portion 200, an air supply portion 300, an air exhaust portion 400, an electric power portion 500, a control portion 600, a display portion 700, an operation portion 800, and an electric power switch 900.

In the pressure detection portion 200, an acoustic filter 210, an electrostatic capacity type pressure sensor 220, and a sensor circuit 230 are arranged. In the air supply portion 300, an automatic pressurization air pump 310 and a pump driving circuit 320 are arranged. In the air exhaust portion 400, a control valve 410 and a control valve driving circuit 420 are arranged. In the electric power portion 500, a battery voltage detection circuit 510 and an electric power circuit 520 are arranged. While, the control portion 600 comprises a micro processor. The display portion 700 comprises a liquid crystal display unit, and the operation portion 800 comprises a pressurization switch.

Herein, as an acoustic filter 210 to be used for removing pressure noise occurring during pressurization of the automatic pressurization air pump 310 used in the electronic tonometer, there may be, for example, one disclosed in Japanese Unexamined Patent Publication No.7-210167 (1995). In the structure of an acoustic filter disclosed in the Publication, there is a plate, and a filter main body portion wherein a capillary portion and a tank portion are arranged in advance so that a capillary and a tank should be formed by the plate being closed, and a packing is pinched inbetween the plate and the filter main body portion, and thereby the plate and the filter main body portion are assembled as a body.

According to this structure, when air including pressure noise is input from the air pressure inlet of the acoustic filter 210, pressure noise is removed by filtration effect by the capillary and the tank, and air pressure is measured by means of the electrostatic capacity type pressure sensor 220 connected to the air pressure outlet of the acoustic filter 210.

However, in the above-mentioned structure of the acoustic filter 210, the structure wherein a packing is inserted into the plate and the filter main body portion is adopted, as a result, uneven thickness of the packing and changes in the thickness owing to changes in ambient temperature cause fluctuations in the amount of the packing encroaching upon the capillary, leading to fluctuated characteristics of the acoustic filter, which has been a problem with the prior art.

### SUMMARY OF THE INVENTION

The present invention has been made so as to solve the problem, and an object of the present invention is to provide an acoustic filter having a structure that does not affect upon the characteristics of an acoustic filter.

According to the present invention, an acoustic filter includes an air pressure inlet, an air pressure outlet connected to the air pressure inlet, and a tank and a capillary arranged in series between the air pressure inlet and the air pressure outlet, the acoustic filter further including a filter case and a filter cap that form the tank and the capillary by being piled up, wherein the filter case and the filter cap have a welding junction structure to be connected by means of welding.

In this structure, wherein there is no arrangement of a packing as seen in the prior art, it becomes possible to stabilize the characteristics of an acoustic filter without giving any influence upon the spatial capacity of a tank and that of a capillary.

And in the invention, it is preferable that the welding conjunction structure has a convex portion arranged on one side of either the filter case or the filter cap, and a concave portion arranged on the other side of either the filter case or the filter cap so as to engage with the concave portion when the filter case and the filter cap are piled up, and the concave portion is melted in the convex portion by means of ultrasonic welding method, thereby the filter case and the filter cap are welded with each other. Thereby, the convex portion is melted in the concave portion, as a consequence, it becomes possible to make secure the air tightness at the welding conjunction portion, -to improve pressure resistance performance against air pressure, and further to stabilize the characteristics of an acoustic filter. Moreover, by use of ultrasonic welding method, it also becomes possible to improve workability of welding of the filter case and the filter cap.

And in the invention, it is preferable that the welding junction structure is arranged so as to encircle the external circumference of the filter case and the filter cap, and further, along the direction wherein the capillary expands, so as to pinch the capillary from both sides thereof, the welding junction structure is arranged inbetween the filter case and the filter cap. Thereby, it is possible to further improve the air tightness in the capillary, and to further enhance the air tightness in the welding conjunction portion.

And in the invention, it is preferable that in the tank, a tank formation portion is arranged on one side of either the filter case or the filter cap, a tank lid portion for closing the tank formation portion is arranged on the other side of either the filter case or the filter cap, and in the capillary, on one side of either the filter case or the filter cap, a capillary formation portion that connects to the tank formation portion when the filter case and the filter cap are piled up is arranged, and a capillary lid portion for closing the capillary formation portion is arranged on the other side of either the filter case or the filter cap.

And in the invention, it is preferable that the acoustic filter is equipped with a first capillary, a first tank, a second capillary, and a second tank, and the first capillary and the second capillary, and the first tank and the second tank are arranged respectively on diagonal line. Since the tanks and capillaries are arranged respectively on diagonal lines, the close adherence of the convex portion as a welding conjunction structure becomes relatively uniform, it becomes possible to weld them evenly. As a result, it is possible to prevent welding failure from occurring.

And in the invention, it is preferable that a ring-shaped packing component is arranged at the air pressure outlet. Thereby, it becomes possible to improve the air tightness in the air pressure outlet.

And in the invention, it is preferable that engaging portions that engage with each other are arranged between the air pressure outlet and the packing component. Thereby, it becomes possible to prevent the packing component from coming out from the air pressure outlet.

And in the invention, it is preferable that a contact material for preventing the packing component from coming in is arranged in the inside of the acoustic filter near the air pressure outlet. Thereby, it becomes possible to prevent the packing component from encroaching into the acoustic filter.

### BRIEF DESCRIPTION OF DRAWINGS

Fig. 1 shows an exploded perspective view of the entire structure of the acoustic filter 1 in the present embodiment.
Fig. 2 shows a top view of the internal structure of the filter case 10 of the acoustic filter 1 in the present embodiment.
Fig. 3 shows a cross sectional view taken along line III-III in Fig. 2.
Fig. 4 shows a partially enlarged view of the area entangled by the circle IV in Fig. 3.
Fig. 5 shows a cross sectional view taken along line V in Fig. 2.
Fig. 6 shows a top view of the internal structure of the filter cap 50 in the present embodiment.
Fig. 7 shows a cross sectional view taken along line VII-VII in Fig. 6.
Fig. 8 shows a partially enlarged view of the area entangled by the circle VIII in Fig. 7.
Fig. 9 shows a cross sectional view taken along line IX-IX in Fig. 6.
Fig. 10 shows an entirely perspective view of the structure of the packing component 80 in the present embodiment.
Fig. 11 shows a cross sectional view taken along line XI-XI in Fig. 10.
Fig. 12 shows a first cross sectional view of the welding conjunction structure of the external circumferential convex portion 11a of the filter case 10 and the external circumferential concave portion 51a of the filter cap 50.
Fig. 13 shows a second cross sectional view of the welding conjunction structure of the external circumferential convex portion 11a of the filter case 10 and the external circumferential concave portion 51a of the filter cap 50.
Fig. 14 shows a first cross sectional view of the welding conjunction structure of the first convex portion 14a of the filter case 10 and the first concave portion 52a of the filter cap 50.
Fig. 15 shows a second cross sectional view of the welding conjunction structure of the first convex portion 14a of the filter case 10 and the second concave portion 52a of the filter cap 50.
Fig. 16 shows acoustic filter frequency characteristics.
Fig. 17 shows the results of the comparison of the cut-off frequencies owing to temperature changes in the acoustic filter 1 according to the present embodiment and the above-mentioned conventional acoustic filter.
Fig. 18 shows a block diagram of the rough structure of an electronic tonometer.

### DESCRIPTION OF THE PREFERRED EMBODIMENTS

The structure of an acoustic filter 1 according to the present invention is explained hereinafter. By the way, the tonometer to which an acoustic filter 1 according to the present embodiment is applied is generally an arm type tonometer, while it may be applied also to a tonometer of other types so long as pressure noise is a problem therein. And the essence of the present invention lies in the structure of acoustic filter 1, therefore, explanation herein is made only about the structure of the acoustic filter 1.

### (Structure of Acoustic filter)

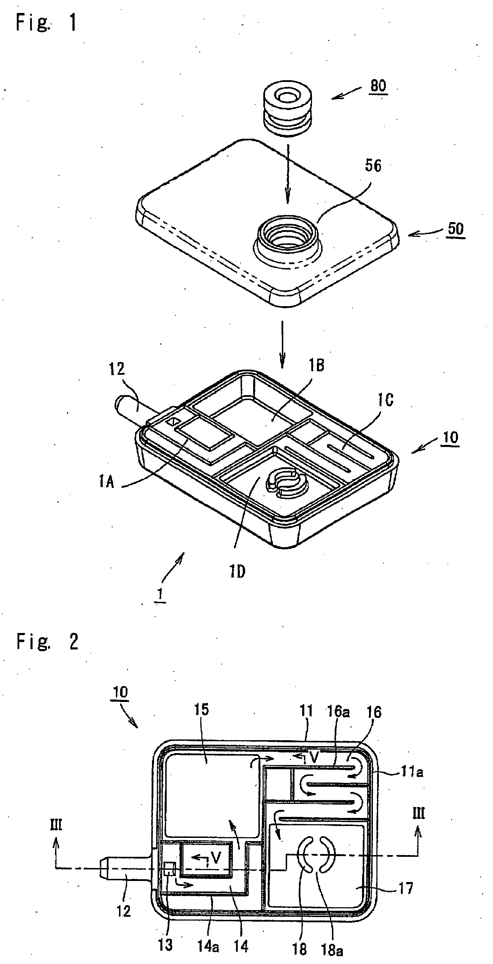

First, with reference to Fig. 1, the structure of an acoustic filter 1 in the present embodiment is explained. By the way, Fig. 1 is an exploded perspective view showing the entire structure of the acoustic filter 1 in the present embodiment.

This acoustic filter 1 is equipped with a filter case 10 having an air pressure inlet 12, a filter cap 50 having an air pressure outlet 56, and a ring-shaped packing component 80 attached to the air pressure outlet 56. The acoustic filter 1 in the present embodiment comprises four portions, and is equipped with a first capillary 1A to which the air pressure inlet 12 directly connects, a first tank 1B that connects to the first capillary 1A, a second capillary 1C that directly connects to the first tank 1B, and a second tank 1D that connects to the second capillary 1C and to which the air pressure outlet 56 directly connects. Therefore, the air pressure inlet 12, the first capillary 1A, the first tank 1B, the second capillary 1C, the second tank 1D, and the air pressure outlet 56 are connected in series. And the first capillary 1A and the second capillary 1C, and the first tank 1B and the second tank 1D are positioned respectively on diagonal lines as shown in the figure.

By the way, in Fig. 1, for clear connection relations, it is shown a status wherein the filter case 10, the filter cap 50, and the packing component 80 are disassembled, however, when used as the acoustic filter 1, the filter case 10, the filter-cap 50, and the packing component 80 are connected in the direction of the arrows in the same figure.

### (Structure of Filter Case 10)

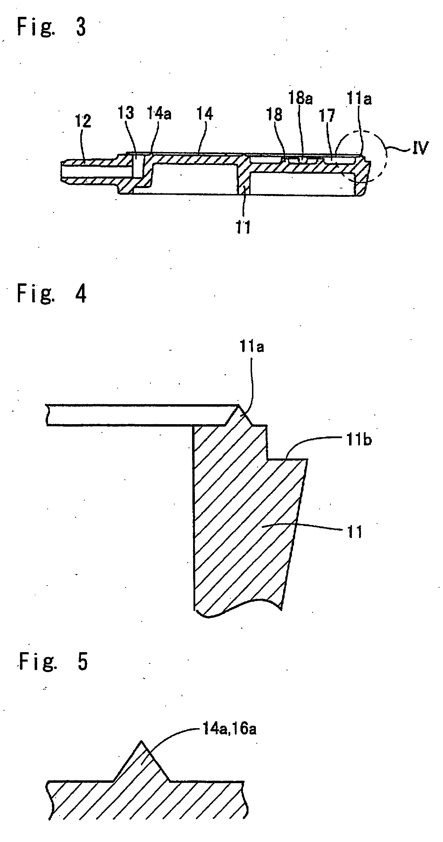

In the next place, the structure of the filter case 10 is explained with reference to Fig. 2 through Fig. 5. By the way, Fig. 2 is a top view showing the internal structure of the filter case 10, Fig. 3 is a cross sectional view at the line III-III in Fig. 2, Fig. 4 is a partially enlarged view of the area entangled by the circle IV in Fig. 3, and Fig. 5 is a cross sectional view at the line V in Fig. 2. And the arrows in Fig. 2 show the air pressure flow in the case when assembled as the acoustic filter 1.

Now with reference to Fig. 2 and Fig. 3, the filter case 10 comprises four portions respectively corresponding to the first capillary 1A, the first tank 1B, the second capillary 1C, and the second tank 1D. First, a case main body portion 11 made of resin, and an external circumferential convex portion 11a are arranged so as to entangle the external circumference of the case main body portion 11. This external circumferential convex portion 11a configures a welding conjunction structure. The shape of the external circumferential convex portion 11a is made into a rough triangle having a convex upward, as shown in the cross sectional view of Fig. 4. And on the external circumferential portion of the external circumferential convex portion 11a, a step 11b is arranged at the position lower than the bottom of the external circumferential convex portion 11a. This step 11b is used for positioning the filter case 10 and the filter cap 50 at the moment of welding.

An air pressure inlet 12 is arranged at the side portion of the case main body portion 11, while an opening portion 12 that connects to the air pressure inlet 12 is arranged at the upper surface portion of the filter case corresponding to the first capillary 1A. Further, a first capillary lid portion 14 is arranged to snake so as to include the opening portion 13, and a first convex portion 14a is arranged so as to regulate the first capillary lid portion 14. This first convex portion 14a configures a welding conjunction structure. While, a first tank formation portion 15 of a plane rectangular shape consisting of specific capacity space is arranged on the upper surface portion of the filter case 10 corresponding to the first tank 1B.

In the next place, a second capillary lid portion 16 is arranged to snake on the upper surface portion of the filter case 10 corresponding to the second capillary 1C, and a second convex portion 16a is arranged so as to regulate the second capillary lid portion 16. This second convex portion 16a configures a welding conjunction structure. While, a second tank formation portion 17 of a plane rectangular shape consisting of specific capacity space is arranged on the upper surface portion of the filter case 10 corresponding to the second tank 1D. At the position opposite to the air pressure outlet 56 of this second tank formation portion 17, a contact material 18 of a rough cylindrical shape is arranged for preventing the packing component 80 from encroaching. By the way, on this contact material 18, a slit 18a is arranged at two positions thereof so as to secure air route.

And the shape of the first convex portion 14a and that of the second convex portion 16a are, as shown in Fig. 5, of a rough triangular shape having a convex upward, just the same as that of the external circumferential convex portion 11a.

### (Structure of Filter Cap 50)

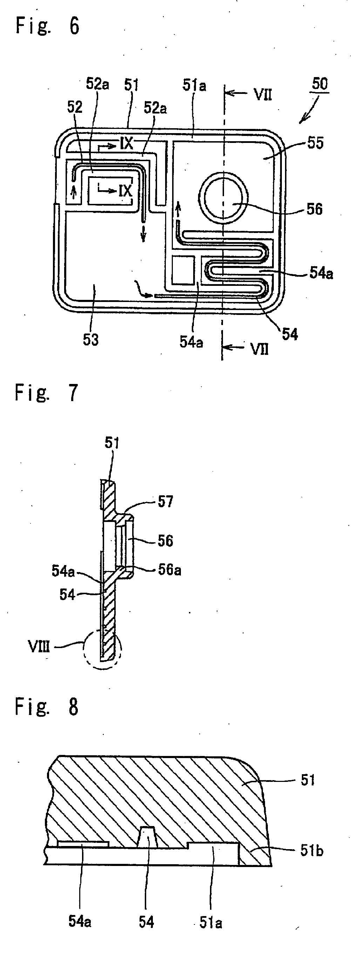

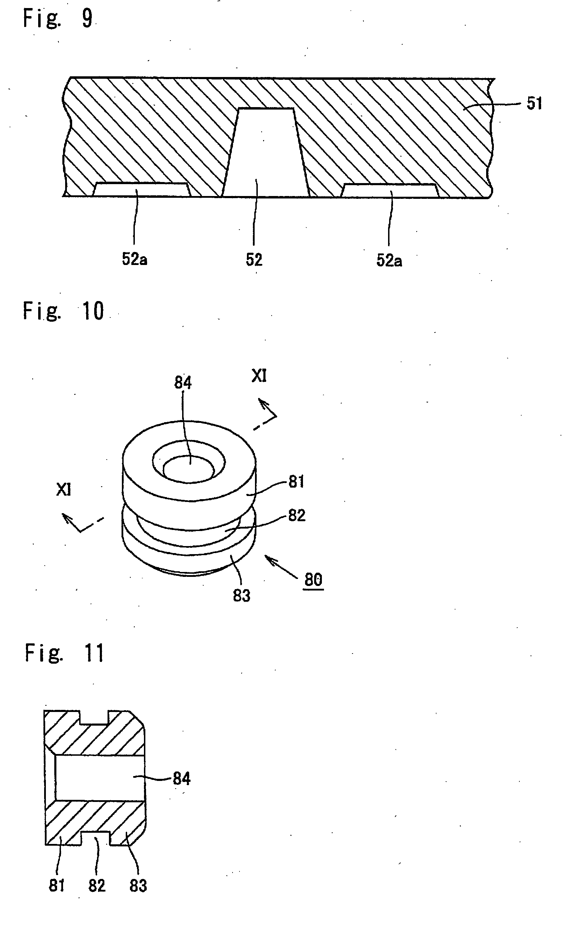

In the next place, the structure of the filter cap 50 is explained hereafter with reference to Fig. 6 through Fig. 9. By the way, Fig. 6 is a top view showing the internal structure of the filter cap 50, Fig. 7 is a cross sectional view at the line VII-VII in Fig. 6, Fig. 8 is a partially enlarged view of the area entangled by the circle VIII in Fig. 7, and Fig. 9 is a cross sectional view at the line IX-IX in Fig. 6. And the arrows in Fig. 2 show the air pressure flow in the case when assembled as the acoustic filter 1.

Now with reference to Fig. 6 and Fig. 7, the filter cap 50 comprises four portions respectively corresponding to the first capillary 1A, the first tank 1B, the second capillary 1C, and the second tank 1D. First, a cap main body portion 51 made of resin, and an external circumferential concave portion 51a are arranged so as to entangle the external circumference of the cap main body portion 51 and at the position opposite to the external circumferential convex portion 11a when piled up. This external circumferential concave portion 51a configures a welding conjunction structure. The shape of the external circumferential concave portion 51a is made into a rough triangle having a concave upward, as shown in the cross sectional view of Fig. 8. And at the external circumferential portion of the external circumferential concave portion 51a, a drooping portion 51b is arranged on the downwardly expanding position to face the step 11b.

On the filter cap 50 corresponding to the first capillary 1A, a first capillary formation portion 52 that is closed by the first capillary lid portion 14 in piled status is arranged to snake, and along the direction of this first capillary formation portion 52 expanding, a first concave portion 52a is arranged at the position to pinch the first capillary formation portion 52 from both sides thereof, and to face the first convex portion 14a. This first concave portion 52a configures a welding conjunction structure. And a first tank lid portion 53 for closing the first tank formation portion 15 is arranged on the upper surface portion of the filter cap 50 corresponding to the first tank 1B.

In the next place, on the filter cap 50 corresponding to the second capillary 1C, a second capillary formation portion 54 that is closed by the second capillary lid portion 16 in piled status is arranged to snake, and along the direction of this second capillary formation portion 54 expanding, a second concave portion 54a is arranged at the position to pinch the second capillary formation portion 54 from both sides thereof, and to face the second convex portion 16a. This second concave portion 54a configures a welding conjunction structure. And a second tank lid portion 55 for closing the second tank formation portion 17 is arranged on the upper surface portion of the filter cap 50 corresponding to the second tank 1D. By the way, on this second tank lid portion 55, an air pressure outlet 56 is arranged. To this air pressure outlet 56, as shown in Fig. 7, a wall portion 57 of a cylindrical shape is arranged, while on the internal circumferential portion of this wall portion 57, a ring-shaped engaging convex portion 56a that extrudes inward is arranged.

And the slot shape of the first capillary formation portion 52 and that of the second capillary formation portion 54, as shown in Fig. 8 and Fig. 9 respectively, have a rough trapezoidal shape having a specific cross sectional capacity as a capillary. While, the slot shape of the first concave portion 52a and that of the second concave portion 54a, as shown in Fig. 8 and Fig. 9 respectively, have a rough rectangular shape , and have a cross sectional capacity enough to accept the first convex portion 14a and the second convex portion 16a at welding.

### (Structure of Packing Component 80)

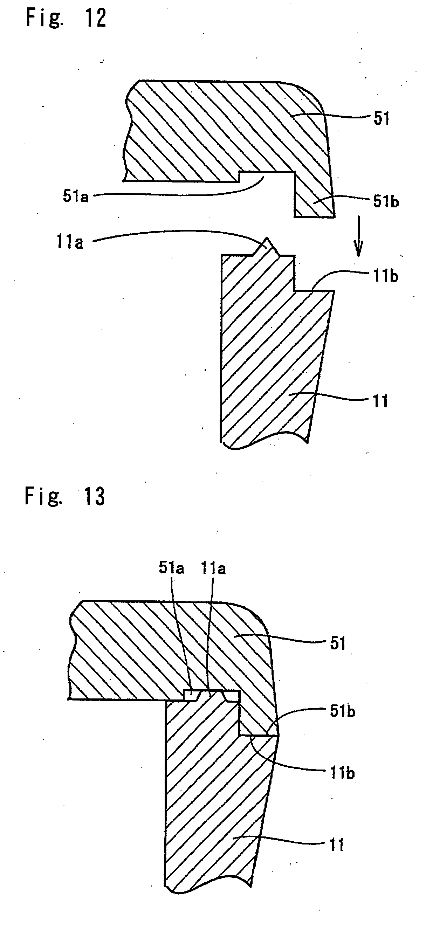

In the next place, the structure of the packing component 80 is explained hereafter with reference to Fig. 10 and Fig. 11. By the way, Fig. 10 is an entirely perspective view showing the structure of the packing component 80, while Fig. 11 is a cross sectional view at the line XI-XI in Fig. 10. This packing component 80 is of a ring shape, and the external circumferential surface consists of an upper ring convex portion 81, an lower ring convex portion 83, and a portion pinched by the upper ring convex portion 81 and the lower ring convex portion 83, and an engaging concave portion 82 to which an engaging convex portion 56a arranged on the air pressure outlet 56 is arranged therein. At the center thereof, a through path 84 expanding in axial direction is arranged.

### (Welding Conjunction Structure)

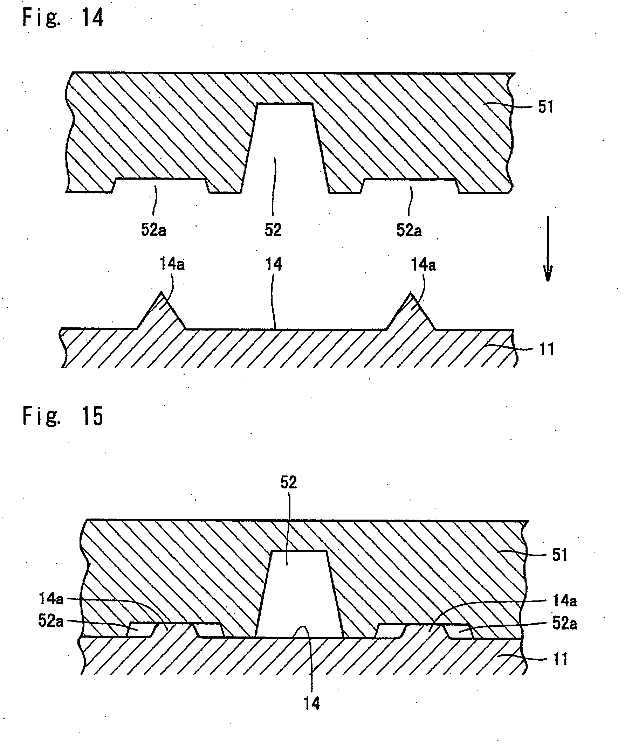

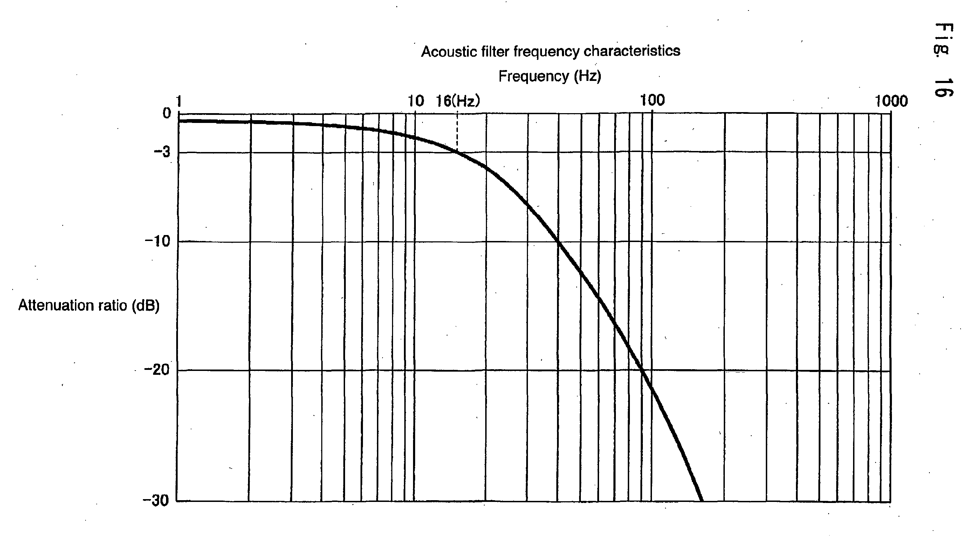

In the next place, the welding conjunction structure in the status wherein the filter case 10 and the filter cap 50 are piled up is explained hereafter with reference to Fig. 12 through Fig. 15. By the way, Fig. 12 and Fig. 13 are cross sectional views showing the welding conjunction structure of the external circumferential convex portion 11a of the filter case 10, and the external circumferential concave portion 51a of the-filter cap 50, while Fig. 14 and Fig. 15 are cross sectional views showing the welding conjunction structure of the first convex portion 14a of the filter case 10, and the second concave portion 54a of the filter cap 50. By the way, the welding conjunction structure of the second convex portion 16a of the filter case 10, and the second concave portion 54a of the filter cap 50 is same as that shown in Fig. 14 and Fig. 15, therefore the explanation thereof is omitted herein.

First, as shown in Fig. 12 and Fig. 13, ultrasonic welding is carried out in the status wherein the external circumferential convex portion 11a and the external circumferential concave portion 51a are piled. Thereby, the external circumferential convex portion 11a is melted in the external circumferential concave portion 51a, and the filter case 10 and the filter cap 50 are welded together.

And as shown in Fig. 14 and Fig. 15, ultrasonic welding is carried out in the status wherein the first convex portion 14a and the first concave portion 52a are piled. Thereby, the first convex portion 14a is melted in the first concave portion 52a, and the filter case 10 and the filter cap 50 are welded together. As a result, the first capillary formation portion 52 is tightly closed by the first capillary lid portion 14, consequently, it is become possible to secure high air tightness of the first capillary.

### (Working Effects)

As mentioned heretofore, in the structure of the acoustic filter 1 according to the present invention, a welding conjunction structure by ultrasonic welding is adopted wherein the concave portions (11a, 14a, and 16a) arranged on the filter case 10 and the concave portions (51a, 52a, and 54a) arranged on the filter cap are mated, it is possible to attain sufficient bonding strength and air tightness in welded portions, and sufficient pressure durability performance against air pressure.

As a result, there is no need for a packing that is subject to uneven thickness arising from production process, and changes in thickness owing to ambient temperature changes as seen in the conventional structures, therefore, it is possible to stabilize the characteristics of the acoustic filter 1 without giving any influence upon the spatial capacity of a tank or that of a capillary.

And the structure according to the present invention has the first capillary 1A, the first tank 1B, the second capillary 1C, and the second tank 1D that are connected in series respectively, and the first capillary 1A and the second capillary 1C, and the first tank 1B and the second tank 1D are arranged on diagonal lines respectively, as a consequence, the close adhesion of the convex portions as part of the welding conjunction structure is relatively even, and it is possible to carry out even welding. As a result, welding failure scarcely occurs.

Further, by arranging a ring-shaped packing component 80 to the air pressure outlet 56, it is possible to enhance the air tightness in the air pressure outlet 56. And by arranging engaging portions (56a and 82) that engage with each other between the air pressure outlet 56 and the packing component 80, it becomes possible to prevent the packing component 80 from coming out from the air pressure outlet 56. Still further, by arranging a contact material 18 to which the packing component 80 contacts in the acoustic filter near the air pressure outlet 56, it becomes possible to prevent the packing component 80 from encroaching into the inside of the acoustic filter 1.

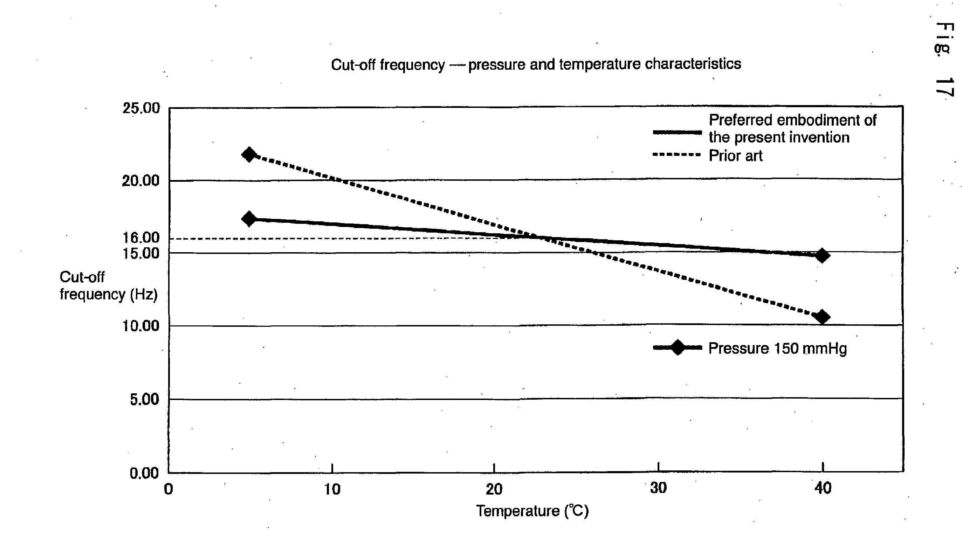

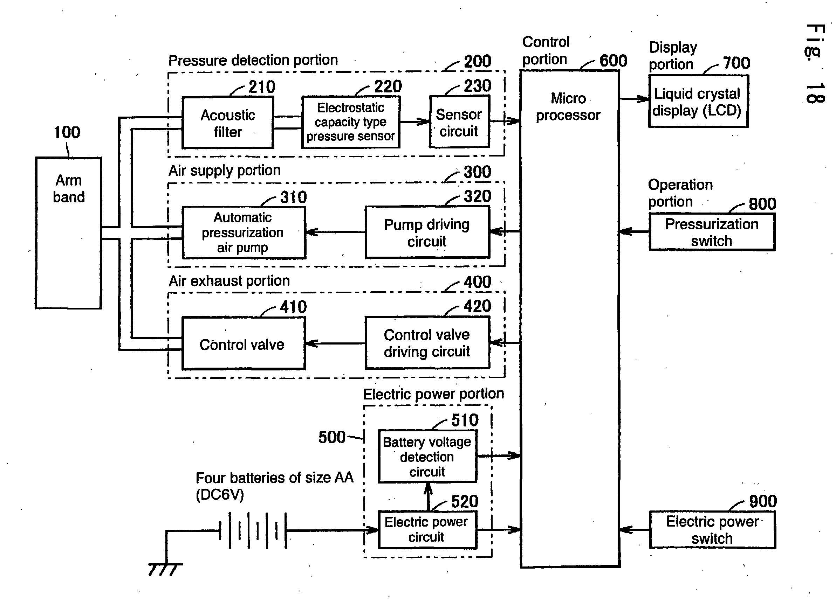

Hereinafter, "Cut-off frequency - pressure and temperature characteristics" in the case of using the acoustic filter according to the embodiment are explained with reference to Fig. 16 and Fig. 17. Fig. 16 shows acoustic filter frequency characteristics, wherein the attenuation ratio (dB) is shown in the vertical axis, while the frequency (Hz) is shown in the horizontal axis by logarithmic divisions. In the acoustic filter 1, the frequency at which the attenuation ratio (dB) is -3 (dB) is defined as cut-off frequency, and the cut-off frequency in this case is approximately 16 (Hz). Fig. 17 shows the results of the comparison of the cut-off frequencies owing to temperature changes in the acoustic filter according to the present embodiment and the above-mentioned conventional acoustic filter, wherein the cut-off frequency 16 Hz is made as reference. By the way, air pressure herein is 150 mmHg.

In Fig. 17, in the case of the acoustic filter of the conventional structure shown in dot line, when ambient temperature goes down, the cut-off frequency goes up significantly, and when ambient temperature goes up, the cut-off frequency goes down greatly. On the other hand, in the case of the acoustic filter according to the present embodiment, there is no conspicuous influence of changes in ambient temperature.

By the way, in the structure according to the present embodiment, as a preferred arrangement of the first capillary 1A, the first tank 1B, the second capillary 1C and the second tank 1D, the case wherein capillaries and tanks are arranged on diagonal lines respectively has been explained' heretofore, however, the present invention is not limited to the arrangement, but it is also possible to adopt a structure wherein capillaries are arranged on one side, and tanks are arranged on the other side.

And, the quantity of capillaries and tanks may be selectively decided at necessity so as to attain required characteristics of an acoustic filter, and is not limited to the quantity mentioned in the embodiment.

Further, the arrangement of capillaries may be varied as a design particular so as to obtain filter effects, and is not limited to the arrangement mentioned in the embodiment.

Still further, in the embodiment, a welding conjunction structure by ultrasonic welding is adopted wherein convex portions arranged on one side and concave portions arranged on the other side are engaged with each other, however, the conjunction method is not limited to this ultrasonic welding, but other conjunction methods may be adopted.

Moreover, tank and capillary lid portions and corresponding tank and capillary formation portions may be arranged respectively to one of the filter case and the filter cap, therefore, their structure is not limited to the structure mentioned in the embodiment.

Therefore, the above-mentioned embodiment disclosed herein is to be considered in all respects as illustrative and not restrictive. The invention may be embodied in other specific forms without departing from the spirit or essential characteristics thereof, and the scope of the invention being indicated by the appended claims rather than by the foregoing description. And all changes which come within the meaning and range of equivalency of the claims are therefore intended to be embraced therein.

According to an acoustic filter under the present invention, wherein a structure without a packing as seen in prior art is adopted, it is possible to stabilize the characteristics of an acoustic filter without giving any influence upon the spatial capacity of a tank and that of a capillary.

## Claims

1. An acoustic filter comprising an air pressure inlet, an air pressure outlet connected to the air pressure inlet, and a tank and a capillary arranged in series inbetween the air pressure inlet and the air pressure outlet, the acoustic filter further having
a filter case and a filter cap that form the tank and the capillary by being piled up, wherein
the filter case and the filter cap have a welding junction structure to be connected by means of welding.

2. An acoustic filter according to claim 1, wherein the welding conjunction structure has a convex portion arranged on one side of either the filter case or the filter cap, and a concave portion arranged on the other side of either the filter case or the filter cap so as to engage with the concave portion when the filter case and the filter cap are piled up, and the convex portion is melted in the concave portion by means of ultrasonic welding method, thereby the filter case and the filter cap are welded with each other.

3. An acoustic filter according to claim 2, wherein the welding junction structure is arranged so as to encircle the external circumference of the filter case and the filter cap, and further, along the direction wherein the capillary expands, so as to pinch the capillary from both sides thereof, the welding junction structure is arranged inbetween the filter case and the filter cap.

4. An acoustic filter according to claim 3, wherein in the tank, a tank formation portion is arranged on one side of either the filter case or the filter cap, and
a tank lid portion for closing the tank formation portion is arranged on the other side of either the filter case or the filter cap, and
in the capillary, on one side of either the filter case or the filter cap, a capillary formation portion that connects to the tank formation portion when the filter case and the filter cap are piled up is arranged, and
a capillary lid portion for closing the capillary formation portion is arranged on the other side of either the filter case or the filter cap.

5. An acoustic filter according to claim 1 to 4, wherein the acoustic filter is equipped with a first capillary, a first tank, a second capillary, and a second tank, and
the first capillary and the second capillary, and the first tank and the second tank are arranged respectively on diagonal lines.

6. An acoustic filter according to claim 1 to 5, wherein a ring-shaped packing component is arranged at the air pressure outlet.

7. An acoustic filter according to claim 1 to 6, wherein engaging portions that engage with each other are arranged between the air pressure outlet and the packing component.

8. An acoustic filter according to claim 1 to 7, wherein a contact material for preventing the packing component from coming in is arranged in the inside of the acoustic filter near the air pressure outlet.
